# EUROPÄISCHE PATENTSCHRIFT

(11) **EP 2 252 340 B1**
(45) Veröffentlichungstag und Bekanntmachung des Hinweises auf die Patenterteilung: **24.08.2016**
(21) Anmeldenummer: 07846552.3
(22) Anmeldetag: 06.11.2007
(51) Int. Cl.: A61L 31/10, A61L 31/14, A61L 31/16

(54) **VERFAHREN ZUR ERZEUGUNG EINER BIOAKTIVEN OBERFLÄCHE AUF EINER ENDOPROTHESE ODER AUF DEM BALLON EINES BALLONKATHETERS**
METHOD FOR PRODUCING A BIOACTIVE SURFACE ON AN ENDOPROSTHESIS OR ON THE BALLOON OF A BALLOON CATHETER
PROCÉDÉ DE PRODUCTION D'UNE SURFACE BIOACTIVE SUR UNE ENDOPROTHÈSE OU SUR LE BALLON D'UN CATHÉTER À BALLON

(43) Veröffentlichungstag der Anmeldung: 24.11.2010
(73) Patentinhaber: Rübben, Alexander, 98000 Monaco (MC)
(72) Erfinder: Rübben, Alexander, 98000 Monaco (MC)
(74) Vertreter: Schöneborn, Holger
(86) Internationale Anmeldenummer: PCT/EP2007/009724
(87) Internationale Veröffentlichungsnummer: WO 2009/059625

(56) Entgegenhaltungen:
- EP-A- 1 329 230
- EP-A- 1 402 849
- WO-A-03/077967
- US-B1- 6 555 157
- US-B1- 6 709 514
- US-B1- 6 723 373
- US-B1- 7 232 490
- J. LAHANN, D. KLEE, H, HÖCKER: "CVD-Beschichtung mit einem funktionalisierten poly-p-xylylen- ein universell anwendbares Verfahren zur Ausrüstung von Medizinimplantaten mit Wirkstoffen" MAT.-WISS. U. WERKSTOFFTECH., Bd. 30, 1999, Seiten 763-766, XP002492390 Weinheim

## Beschreibung

Die vorliegende Erfindung betrifft ein Verfahren zur Erzeugung einer bioaktiven Oberfläche auf einer Endoprothese oder auf dem Ballon eines Ballonkatheters.

Die sogenannten "minimalinvasiven Verfahren" nehmen in der Medizin einen immer größeren Stellenwert ein. Im Rahmen der Radiologie ist hierbei die interventionelle Radiologie anzusprechen, die wesentlich zur Entwicklung minimalinvasiver Techniken und hierfür notwendiger Geräte und Prothesen aus geeigneten Materialien beigetragen hat. So werden heute kleine Metallgitter als Gefäßendoprothesen, sogenannte Stents, sowohl von Kardiologen als auch von Radiologen in Gefäße eingesetzt, um diese offen zu halten. Bei herkömmlichen Stents kommt es jedoch häufig zu einer Verdickung der Gefäßwand mit konsekutiver Lumeneinengung im Bereich des Stents durch eine Zellproliferation oder durch eine Anlagerung von Zellen. Weiterhin werden Ballonkatheter sowohl von Kardiologen als auch von Radiologen in Gefäße eingesetzt, um diese zu öffnen. Auch bei diesen Eingriffen kann es zu einer Verdickung der Gefäßwand mit konsekutiver Lumeneinengung im Bereich der Aufdehnung durch eine Zellproliferation kommen.

Durch Medikamentenabgabe von der Oberfläche der Endoprothese beziehungsweise von der Oberfläche des Ballons des Ballonkatheters, die zur Verbesserung der Medikamentenbeladung und Medikamentenabgabe mit einer geeigneten polymeren Beschichtung versehen sein kann, kann diesem Problem entgegengewirkt werden. Typischerweise wird ein in einem Lösungsmittel gelöster Wirkstoff auf die Oberfläche der Endoprothese oder auf die Oberfläche des Ballons des Ballonkatheters aufgebracht, wobei das Lösungsmittel anschließend verdunstet. Dieses Verfahren birgt allerdings das Risiko, dass infolge einer möglicherweise unregelmäßigen Verteilung des Lösungsmittels und damit auch des Wirkstoffes die Wirkstoffbeschichtung unregelmäßig erfolgt. Das bedeutet, dass es möglicherweise zu einer Wirkstoffklumpenbildung kommen kann, also zu einer unregelmäßigen Verteilung des Wirkstoffes auf der Oberfläche der Endoprothese beziehungsweise des Ballons des Ballonkatheters.

Es ist daher die Aufgabe der vorliegenden Erfindung, ein vorteilhaftes Verfahren zur Erzeugung einer bioaktiven Oberfläche auf einer Endoprothese oder auf dem Ballon eines Ballonkatheters zur Verfügung zu stellen. Diese Aufgabe wird durch ein Verfahren nach Anspruch 1 gelöst. Die abhängigen Ansprüche enthalten weitere vorteilhafte Ausgestaltungen der Erfindung.

In dem erfindungsgemäßen Verfahren zur Erzeugung einer bioaktiven Oberfläche auf einer Endoprothese oder auf dem Ballon eines Ballonkatheters wird zunächst die Oberfläche der Endoprothese bzw. die Oberfläche des Ballons mit einer Lösung eines Wirkstoffes benetzt. Anschließend wird überflüssiger Wirkstoff und Lösungsmittel durch Bewegen der Endoprothese bzw. des Ballons von der benetzten Oberfläche entfernt. Danach wird das Lösungsmittel von dem Wirkstoff getrennt. Vorteilhafterweise wird überflüssiger Wirkstoff und Lösungsmittel durch Abschleudern oder Abschlagen von der benetzten Oberfläche entfernt. Durch das Bewegen der Endoprothese bzw. des Ballons, insbesondere durch Schleudern, wird eine gleichmäßige Verteilung des Lösungsmittels und damit auch des Wirkstoffes auf der Oberfläche der Endoprothese bzw. des Ballons erreicht. Dies beugt einer möglichen Bildung von Klumpen effektiv vor.

Weiterhin kann die Oberfläche der Endoprothese bzw. des Ballons vor dem Benetzen vergrößert werden. Beispielsweise kann die Oberfläche der Endoprothese bzw. des Ballons mechanisch, thermisch oder chemisch vergrößert werden. Die Oberfläche der Endoprothese bzw. des Ballons kann insbesondere durch Strukturieren oder Profilieren vergrößert werden. Beispielsweise kann die Oberfläche der Endoprothese bzw. des Ballons durch Aufrauen strukturiert oder profiliert werden. Vorteilhafterweise werden durch das Vergrößern der Oberfläche der Endoprothese oder des Ballons auf der Oberfläche Vertiefungen mit einer Tiefe von 5-50 µm und einer Breite von 5-50 µm erzeugt.

Der verwendete Wirkstoff kann insbesondere Tretinoin und/oder Tretinoinderivate und/oder Orphanrezeptoragonisten und/oder Elafinderivate und/oder Corticosteroide und/oder Steroidhormone und/oder Paclitaxel und/oder Taxol und/oder Taxolderivate und/oder Rapamune und/oder Tacrolimus und/oder hydrophobe Proteine und/oder zellproliferationsverändernde Substanzen umfassen. Als Steroidhormone können beispielsweise Methylprednisolon, Dexamethason oder Östradiol verwendet werden.

Als Lösungsmittel kann beispielsweise Dimethylsulfoxid (DMSO), Dioxan, Dimethylformamid (DMF) oder Tetrahydrofuran (THF) verwendet werden. Diese Lösungsmittel sind insbesondere mit Wasser mischbar. Die Oberfläche der Endoprothese bzw. des Ballons kann beispielsweise durch Eintauchen, Besprühen oder Pipettieren benetzt werden.

Die Oberfläche der Endoprothese oder die Oberfläche des Ballons ist mit einer Lösung eines Wirkstoffes in einem mit Wasser mischbaren Lösungsmittel benetzt, wobei der Wirkstoff eine Löslichkeit von maximal 0,9 mg/ml in destilliertem Wasser hat. In diesem Fall kann das Lösungsmittel von dem Wirkstoff getrennt werden, indem die benetzte Endoprothese oder der benetzte Ballon in Wasser getaucht wird. Dabei fällt der in Wasser unlösliche Wirkstoff aus und scheidet sich teilweise auf der Oberfläche der Endoprothese bzw. des Ballons ab. Als wasserlösliche Lösungsmittel können insbesondere die oben genannten Lösungsmittel Verwendung finden. Alternativ dazu kann das Lösungsmittel von dem Wirkstoff auch durch Verflüchtigenlassen des Lösungsmittels getrennt werden. In diesem Fall ist eine Wasserlöslichkeit des Lösungsmittels nicht erforderlich.

Grundsätzlich kann die Oberfläche der Endoprothese bzw. des Ballons vor der Erzeugung der bioaktiven Oberfläche mit einer Polymerschicht funktionell beschichtet werden. In diesem Fall kann die Dicke der funktionellen Polymerschicht 10-1000 µm, vorteilhafterweise 200-400 µm, betragen. Die Oberfläche der Endoprothese bzw. des Ballons kann vor der Erzeugung der bioaktiven Oberfläche beispielsweise mit Polyamino-p-xylylen-co-polyxylylen funktionell beschichtet werden.

Zur Herstellung einer funktionellen Polymerschicht können aus den Ausgangsverbindungen der allgemeinen Strukturen (1), (2) und/oder (3) bei erhöhten Temperaturen und reduzierten Drücken Monomere in der Gasphase erzeugt und durch Abkühlung bei reduzierter Temperatur polymerisiert werden, mit: 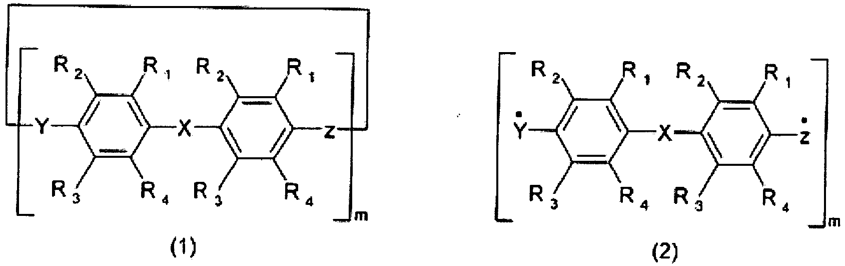 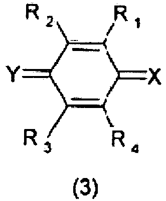
R_{1,2,3,4}: jeweils, gleich oder verschieden voneinander, Wasserstoffatome, Halogenatome, Alkylgruppen oder substituierte Alkylgruppen, Arylgruppen oder substituierte Arylgruppen, organische Reste oder Radikale, Gruppen der allgemeinen Struktur CO(O-M-A), metallierte Gruppen, Hydroxylgruppen, Aminogruppen, Carboxylgruppen, Estergruppen, Ethergruppen, Säurehalogenidgruppen, Isocyanatgruppen, schwefelhaltige Gruppen, stickstoffhaltige Gruppen, phosphorhaltige Gruppen, siliziumhaltige Gruppen; X,Y: Kohlenwasserstoffreste
m: Zahl der Wiederholungseinheiten = 1-20
wobei die zur Herstellung der Monomere benötigten Temperaturen zwischen 500°C und 1000°C und die benötigten Drücke kleiner als 500 Pa sind. Dimere der Struktur (1) oder (2) mit m=1 können beispielsweise bei Temperaturen zwischen 600°C und 900°C und Drücken kleiner als 100 Pa zu Monomeren gespalten werden. Die anschließende Polymerisation kann dann bei Temperaturen unter 120°C durchgeführt werden.

Grundsätzlich kann im Rahmen des erfindungsgemäßen Verfahrens als Endoprothese ein Stent verwendet werden.

Der Vorteil des erfindungsgemäßen Verfahrens besteht darin, dass durch das Bewegen der Endoprothese bzw. des Ballons, insbesondere durch Abschleudern oder Abschlagen, eine gleichmäßige Verteilung des den Wirkstoff enthaltenden Lösungsmittels auf der Oberfläche erzielt wird. Dadurch kann einer möglichen Klumpenbildung des Wirkstoffes wirksam vorgebeugt werden.

Weitere Merkmale, Eigenschaften und Vorteile der vorliegenden Erfindung werden nachfolgend anhand von Ausführungsbeispielen unter Bezugnahme auf die beiliegenden Figuren beschrieben.
Fig. 1 zeigt schematisch einen Ballonkatheter, der mit der Lösung eines Wirkstoffes besprüht wird.
Fig. 2 zeigt schematisch einen Ballonkatheter, auf dessen Oberfläche sich die Lösung eines Wirkstoffes befindet.
Fig. 3 zeigt schematisch das Abschleudern von überflüssigem Wirkstoff und überflüssigem Lösungsmittel von dem Ballon eines Ballonkatheters.
Fig. 4 zeigt schematisch das Abschlagen von überflüssigem Wirkstoff und überflüssigem Lösungsmittel von dem Ballon eines Ballonkatheters.
Fig. 5 zeigt schematisch einen Schnitt durch einen Teil der Oberfläche eines Ballonkatheters.
Fig. 6 zeigt schematisch das Eintauchen eines mit der Lösung eines Wirkstoffes benetzten Ballonkatheters in ein Wasserbad.
Fig. 7 zeigt schematisch den Schnitt durch einen Teil der Oberfläche eines Ballonkatheters, der sich im Wasserbad befindet und auf dessen Oberfläche Wirkstoff ausfällt.
Fig. 8 zeigt schematisch einen Schnitt durch einen Teil einer Oberfläche eines Ballonkatheters, an die sich Wirkstoff angelagert hat.
Fig. 9 zeigt schematisch einen Schnitt durch einen Teil der Oberfläche eines Ballonkatheters, die mit einem Polymer funktionell beschichtet wurde und deren aufgeraute Polymerschicht weiterhin mit einem Wirkstoff beschichtet wurde.
Fig. 10 zeigt schematisch den Schnitt durch einen Teil eines Gittergerüstes eines Stents, dessen Oberfläche zunächst mit einem Polymer funktionell beschichtet und anschließend mit einer Lösung eines Wirkstoffes benetzt wurde.
Fig. 11 zeigt schematisch den in der Figur 9 gezeigten Schnitt, nachdem überschüssiger Wirkstoff und Lösungsmittel von der Oberfläche des Stents entfernt wurden.
Fig. 12 zeigt schematisch den in der Figur 10 gezeigten Schnitt durch einen Teil des Gittergerüstes eines Stents, nachdem das Lösungsmittel von dem Wirkstoff getrennt wurde.
Fig. 13 zeigt einen Schnitt durch einen Teil eines Gittergerüstes eines Stents, der lediglich mit einem Wirkstoff beschichtet wurde.
Fig. 14 zeigt schematisch einen Schnitt durch einen Teil eines Gittergerüstes eines Stents, wobei die Oberfläche des Stents zunächst aufgeraut und anschließend mit einem Wirkstoff beschichtet wurde.

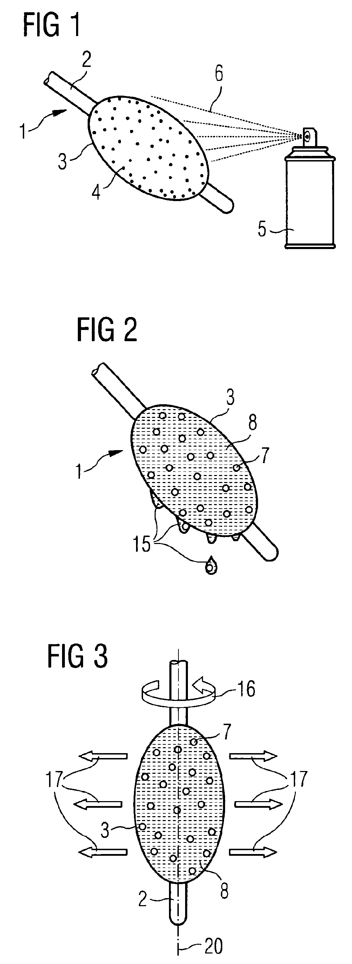

Ein erstes Ausführungsbeispiel der vorliegenden Erfindung wird nachfolgend anhand der Figuren 1 bis 7 näher beschrieben. Die Figur 1 zeigt schematisch einen Ballonkatheter 1, der mithilfe einer Sprühvorrichtung 5 mit der Lösung 6 eines Wirkstoffes 7 in einem mit Wasser mischbaren Lösungsmittel 8 besprüht wird. Der Ballonkatheter 1 umfasst eine Kathetersonde 2 und einen Ballon 3. Der Ballon 3 umschließt einen Teil der Kathetersonde 2. Die Oberfläche des Ballons 3 wurde zunächst durch Aufrauen vergrößert. Dazu wurde die Oberfläche des Ballons 3 beispielsweise mechanisch, thermisch oder chemisch aufgeraut. Die durch das Aufrauen auf der Oberfläche entstandenen Vertiefungen sind in der Figur 1 schematisch durch mit der Bezugsziffer 4 gekennzeichnete Punkte angedeutet. Anschließend wird die so aufgeraute Oberfläche 4 des Ballons 3 mithilfe einer Sprühvorrichtung 5 mit einer Lösung 6 eines Wirkstoffes 7 in einem mit Wasser mischbaren Lösungsmittel 8 besprüht. Anstelle des Besprühens kann der Ballon 3 des Ballonkatheters 1 auch in die Lösung eines Wirkstoffes in einem mit Wasser mischbaren Lösungsmittel eingetaucht oder mit dieser pipettiert werden.

Die Figur 2 zeigt schematisch den Ballonkatheter 1, dessen Ballon 3 auf der Oberfläche mit der Lösung eines Wirkstoffes 7 auf diese Weise benetzt wurde. Auf der zunächst aufgerauten Oberfläche des Ballons 3 befindet sich nun eine Mischung aus einem Lösungsmittel 8 und einem Wirkstoff 7. Bei dem verwendeten Wirkstoff 7 kann es sich beispielsweise um Tretinoin und/oder Tretinoinderivate und/oder Orphanrezeptoragonisten und/oder Elafinderivate und/oder Corticosteroide und/oder Steroidhormone und/oder Paclitaxel und/oder Taxol und/oder Taxolderivate und/oder Rapamune und/oder Tacrolimus und/oder hydrophobe Proteine und/oder zellproliferationsverändernde Substanzen handeln, wobei als Steroidhormone Methylprednisolon, Dexamethason oder Östradiol verwendet werden können. Bei dem Lösungsmittel 8 kann es sich beispielsweise um Dimethylsulfoxid (DMSO), Dioxan, Dimethylformamid (DMF) oder Tetrahydrofuran (THF) handeln.

Infolge des Benetzungsprozesses ist die Oberfläche des Ballons 3 nicht gleichmäßig mit Wirkstoff 7 und Lösungsmittel 8 bedeckt. Dies ist in der Figur 2 in Form von sich bildenden Tropfen 15, die überflüssigen Wirkstoff und überflüssiges Lösungsmittel kennzeichnen, angedeutet. Zur Vermeidung einer daraus resultierenden späteren Klumpenbildung auf der Oberfläche des Ballons 3 wird der Ballonkatheter 1 oder zumindest der Ballon 3 so bewegt, dass sich überschüssiger Wirkstoff 7 und überschüssiges Lösungsmittel 8 von der Oberfläche ablösen. Dies ist schematisch in der Figur 3 dargestellt.

In der Figur 3 ist gezeigt, wie der Ballonkatheter 1 in eine schnelle Rotation versetzt, also geschleudert wird. Die Rotationsbewegung ist durch einen Pfeil 16 gekennzeichnet. Infolge des Schleuderns lösen sich überschüssiges Lösungsmittel 8 und überschüssiger Wirkstoff 7 von der Oberfläche des Ballons 3 aufgrund der auf sie wirkenden Zentrifugalkraft ab. Dies ist durch Pfeile 17 gekennzeichnet, die die Richtung der auf den überschüssigen Wirkstoff 7 und das überschüssige Lösungsmittel 8 wirkenden, in Bezug auf die Drehachse 20 radial nach außen gerichteten Zentrifugalkraft andeuten.

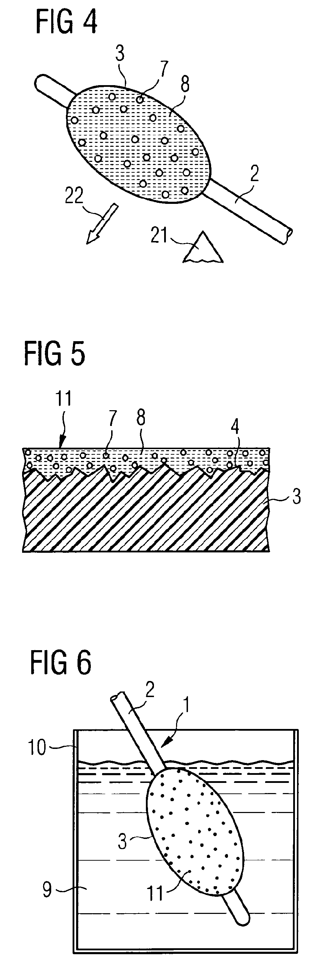

Alternativ oder kumulativ dazu kann die überschüssiges Lösungsmittel 8 und überschüssiger Wirkstoff 7 auch durch Abschlagen von dem Ballon 3 entfernt werden. Dabei wird der Ballonkatheter 1 vorsichtig gegen einen Anschlag 21 geschlagen, so dass sich überschüssiges Lösungsmittel 8 und überschüssiger Wirkstoff 7 aufgrund der beim Abschlagen entstehenden Beschleunigungskräfte vom Ballon 3 ablösen. Dies ist schematisch in der Figur 4 gezeigt. Die Bewegungsrichtung des Ballonkatheters 1 beim Abschlagen ist durch einen Pfeil 22 gekennzeichnet.

Eine weitere Variante besteht darin, den Ballonkatheter 1 oder zumindest den Ballon 3 in eine Vibration zu versetzen und dadurch ein Ablösen des überschüssigen Lösungsmittels 8 und des überschüssigen Wirkstoffes 7 zu erzielen.

In der Figur 5 ist schematisch ein Schnitt durch einen Teil der Oberfläche des Ballons 3 des Ballonkatheters 1 nach Durchführung des Schleuderns oder Abschlagens dargestellt. Die aufgeraute Ballonoberfläche 4 ist dann gleichmäßig mit einer Schicht bestehend aus dem verwendeten Wirkstoff 7 und dem Lösungsmittel 8 benetzt. Die so entstandene aufgeraute und benetzte Oberfläche 11 zeichnet sich dadurch aus, dass sie keine Tropfen 15 oder andere Ansammlungen von überflüssigem Wirkstoff 7 und überflüssigem Lösungsmittel 8 aufweist.

Anschließend wird der nun gleichmäßig benetzte Ballon 3 des Ballonkatheters 1 in ein Wasserbad eingetaucht. Dies ist schematisch in Figur 6 dargestellt. Die Figur 6 zeigt ein Gefäß 10, welches mit Wasser 9 gefüllt ist. Der Ballon 3 des Ballonkatheters 1 ist vollständig in das Wasser 9 eingetaucht. Die aufgeraute und benetzte Oberfläche 11 des Ballons 3 kommt dabei direkt mit dem Wasser 9 in Kontakt.

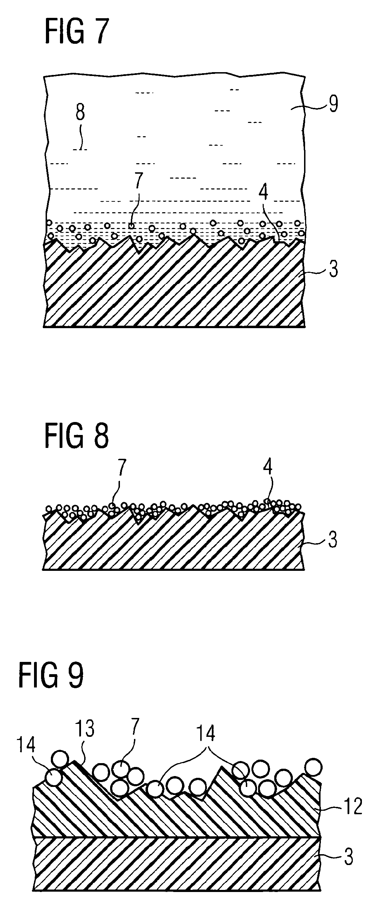

Die Figur 7 zeigt schematisch einen Schnitt durch einen Teil der sich im Wasser 9 befindlichen Oberfläche 4 des Ballons 3 des Ballonkatheters 1. Infolge des direkten Kontaktes zwischen dem Wasser 9 und dem mit Wasser mischbaren Lösungsmittel 8 tritt eine zunehmende Vermischung des Lösungsmittels 8 mit dem Wasser 9 auf. Dies ist in der Figur 7 schematisch angedeutet. Der im Wasser unlösliche oder zumindest nur schwer lösliche Wirkstoff 7 fällt hingegen auf der aufgerauten Oberfläche 4 des Ballons 3 aus. Auf diese Weise wird der Wirkstoff 7 von dem Lösungsmittel 8 getrennt.

Nachdem der Wirkstoff 7 auf der Oberfläche 4 ausgefallen ist, kann der Ballon 3 des Ballonkatheters 1 aus dem Wasserbad entfernt werden. Das möglicherweise auf der Oberfläche 4 des Ballons 3 noch befindliche Wasser kann anschließend verdunstet werden. Die Figur 8 zeigt schematisch die durch das Verfahren erzielte Beschichtung der Oberfläche 4 des Ballons 3. In der Figur 8 ist ein Schnitt durch einen Teil der aufgerauten Oberfläche 4 des Ballons 3 des Ballonkatheters 1 gezeigt. Auf der aufgerauten Ballonoberfläche 4 befindet sich nun der ausgefällte Wirkstoff 7 in Form einer gleichmäßigen Beschichtung. Die Beschichtung weist insbesondere keine Verklumpungen auf.

Im Folgenden wird ein zweites Ausführungsbeispiel anhand der Figur 9 näher beschrieben. Elemente, die solchen Elementen entsprechen, die bereits im ersten Ausführungsbeispiel beschrieben wurden, sind mit denselben Bezugsziffern versehen und werden nicht erneut beschrieben. Das vorliegende Ausführungsbeispiel bezieht sich ebenfalls auf einen Ballon 3 eines Ballonkatheters 1, der mit einem Wirkstoff 7 beschichtet werden soll. Im Unterschied zum ersten Ausführungsbeispiel wird der Ballon 3 des Ballonkatheters 1 zunächst mit einer Polymerschicht 12 funktionell beschichtet. Dazu wird beispielsweise auf den Ballon 3 des Ballonkatheters 1 das dimere 4-Amino-[2,2]-paracyclophan bei 700°C und 20 Pa in reaktive Monomere gespalten und polymerisiert anschließend auf der auf etwa 20°C gekühlten Oberfläche des Ballons 3. Die angestrebte Dicke der Polymerbeschichtung beträgt vorteilhaft 10-1000 µm, noch vorteilhafter 200-400 µm.

Die so erzeugte Polymerschicht 12 kann nun entweder direkt mit einer Lösung 6 eines Wirkstoffes 7 in einem mit Wasser mischbaren Lösungsmittel 8, wie im ersten Ausführungsbeispiel anhand der Figur 1 beschrieben wurde, benetzt werden. Alternativ dazu kann die Oberfläche der Polymerbeschichtung 12 vor dem Benetzten zunächst beispielsweise durch Aufrauen vergrößert werden. Die im ersten Ausführungsbeispiel näher beschriebenen Möglichkeiten zur Vergrößerung der Oberfläche des Ballons 3 sind für eine mögliche Vergrößerung der Oberfläche der Polymerbeschichtung 12 im vorliegenden Ausführungsbeispiel ebenfalls anwendbar. Die aufgeraute Oberfläche der Polymerbeschichtung 12 wird anschließend, wie im ersten Ausführungsbeispiel beschrieben wurde, mit einer Lösung 6 eines Wirkstoffes 7 in einem mit Wasser mischbaren Lösungsmittel 8 benetzt. Als Lösungsmittel 8 beziehungsweise Wirkstoff 7 können die im ersten Ausführungsbeispiel genannten Substanzen verwendet werden.

Nach dem Benetzen der entweder glatten oder aber aufgerauten Oberfläche des polymerbeschichteten Ballons 3 wird überschüssiger Wirkstoff 7 und überschüssiges Lösungsmittel 8 durch ein schnelles Bewegen des Ballons 3, beispielsweise durch Schleudern oder Schütteln, von der Oberfläche entfernt. Beispielsweise kann der überschüssige Wirkstoff 7 und das überschüssige Lösungsmittel 8 von der Oberfläche auch abgeschlagen werden.

Danach kann wahlweise das auf der Oberfläche befindliche Lösungsmittel 8 verdunsten gelassen werden oder es kann der Ballon 3, wie im ersten Ausführungsbeispiel anhand der Figur 6 beschrieben wurde, in ein Wasserbad getaucht werden, wobei der Wirkstoff 7 auf der polymerbeschichteten Oberfläche 13 ausfällt. Bei einem Verdunstenlassen ist auch die Verwendung eines nicht oder nur schwer wasserlöslichen Lösungsmittels möglich.

Die auf die beschriebene Weise erzielte Beschichtung ist schematisch in der Figur 9 dargestellt. Die Figur 9 zeigt einen Schnitt durch einen Teil der Oberfläche des Ballons 3 des Ballonkatheters 1. Auf der Oberfläche des Ballons 3 befindet sich eine Polymerbeschichtung 12. Die Oberfläche 13 der Polymerbeschichtung 12 ist vor dem Benetzen mit einer Lösung eines Wirkstoffes 7 zunächst aufgeraut worden. Nach dem Abschluss des Ausfällens beziehungsweise nach dem das Lösungsmittels verdunstengelassen wurde hat sich der Wirkstoff 7 auf der Oberfläche 13 der Polymerschicht 12 gleichmäßig abgelagert. Ein Teil des Wirkstoffes 7 hat sich zudem in die Polymerschicht 12 eingelagert. Dieser Teil des Wirkstoffes ist mit der Bezugsziffer 14 gekennzeichnet.

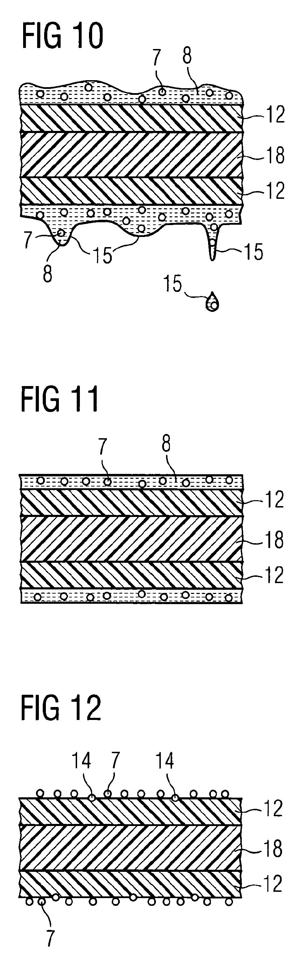

Es wird nun ein drittes Ausführungsbeispiel anhand der Figuren 10 bis 12 näher beschrieben. Das vorliegende Ausführungsbeispiel behandelt die erfindungsgemäße Beschichtung eines Stents mit einem Wirkstoff. Elemente, die bereits im Zusammenhang mit den vorangegangenen Ausführungsbeispielen beschrieben wurden, sind mit denselben Bezugsziffern gekennzeichnet und werden nicht erneut im Detail beschrieben.

Die Figur 10 zeigt schematisch einen Schnitt durch einen Teil eines Gittergerüstes eines Stents 18. Die Oberfläche des Stents 18 ist zunächst, wie im zweiten Ausführungsbeispiel näher beschrieben wurde, funktionell mit einer Polymerschicht 12 beschichtet worden. Die Oberfläche dieser Polymerbeschichtung 12 wurde anschließend, wie in den vorangegangenen Ausführungsbeispielen bereits beschrieben, mit einer Lösung eines Wirkstoffes 7 in einem mit Wasser mischbaren Lösungsmittel 8 benetzt. Als Wirkstoff 7 und als Lösungsmittel 8 können die im ersten Ausführungsbeispiel genannten Substanzen verwendet werden. Typischerweise treten infolge der Benetzung auf der Oberfläche Unebenheiten oder Tropfen 15 aus Lösungsmittel 8 und Wirkstoff 7 auf. Dies ist in der Figur 10 schematisch angedeutet. Eine erhöhte Tropfenbildung oder eine erhöhte Ansammlung der Wirkstofflösung findet insbesondere dort statt, wo sich Streben des Stentgitters berühren, also an Kontakt- und Kreuzungsstellen. Zur Vermeidung einer späteren Verklumpung des Wirkstoffes 7 auf der Oberfläche infolge einer Ansammlung oder infolge von Unebenheiten beziehungsweise Tropfenbildung wird der überschüssige Wirkstoff 7 und das überschüssige Lösungsmittel 8 durch schnelles Bewegen, Schleudern oder Abschlagen von der Oberfläche des Stents 18 entfernt. Zu weiteren Einzelheiten wird hierbei auf die vorangegangenen Ausführungsbeispiele verwiesen.

Nachdem überschüssiger Wirkstoff 7 und überschüssiges Lösungsmittel 8 von der Oberfläche entfernt wurden, weist die polymerbeschichtete Oberfläche des Stents 18 nun eine deutlich gleichmäßigere Benetzung mit Wirkstoff 7 und Lösungsmittel 8 auf. Dies ist schematisch in der Figur 11 dargestellt. Die Figur 11 zeigt den bereits in der Figur 10 dargestellten Schnitt, wobei die Oberfläche der Polymerschicht 12 nun weitgehend gleichmäßig mit einem Wirkstoff 7 und einem Lösungsmittel 8 benetzt ist.

Nachfolgend kann entweder das Lösungsmittel 8 verdunstet werden, wobei das verwendete Lösungsmittel 8 wasserlöslich oder nicht wasserlöslich sein kann. Alternativ dazu kann der Stent 18, wie im ersten Ausführungsbeispiel im Zusammenhang mit der Figur 6 anhand des Ballonkatheters 1 beschrieben wurde, in ein Wasserbad getaucht werden, wobei der Wirkstoff 7 auf der Oberfläche der Polymerschicht 12 ausfällt. Das in beiden Varianten erzielte Ergebnis ist in der Figur 12 schematisch dargestellt. Die Figur 12 zeigt den bereits in den Figuren 10 und 11 dargestellten Schnitt durch einen Teil eines Gittergerüstes eines Stents 18. Auf der Oberfläche des Stents 18 befindet sich eine Polymerbeschichtung 12. Auf der Oberfläche der Polymerbeschichtung 12 hat sich der Wirkstoff 7 gleichmäßig abgelagert. Ein Teil des Wirkstoffes 7 hat sich in die Polymerschicht 12 eingelagert. Dieser Teil des Wirkstoffes ist mit der Bezugsziffer 14 gekennzeichnet.

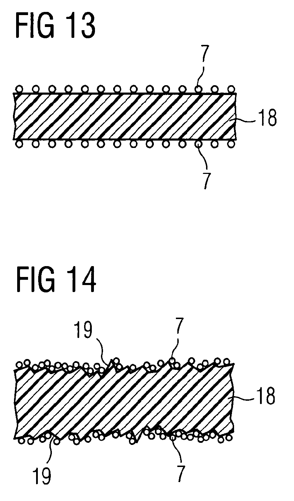

Mithilfe des beschriebenen Verfahrens lässt sich selbstverständlich auch die Oberfläche eines Stents 18, der keine Polymerbeschichtung 12 aufweist, mit einem Wirkstoff 7 beschichten. Dies soll im Folgenden anhand der Figuren 13 und 14 näher beschrieben werden. Elemente, die Elementen der vorangegangenen Ausführungsbeispiele entsprechen, sind mit denselben Bezugsziffern versehen und werden nicht erneut in Detail beschrieben. Weiterhin wird auch in Bezug auf die Einzelheiten und möglichen Ausführungsvarianten des erfindungsgemäßen Verfahrens auf die vorangegangenen Ausführungsbeispiele verwiesen.

Die Oberfläche des Stents 18 wurde zunächst mit einer Lösung eines Wirkstoffes 7 in einem mit Wasser mischbaren Lösungsmittel 8 benetzt. Anschließend wurde überschüssiger Wirkstoff 7 und überschüssiges Lösungsmittel 8 von der Oberfläche des Stents 18 durch schnelles Bewegen, Schleudern des Stents 18 oder Abschlagen von der Oberfläche des Stents 18 entfernt. Danach wurde das Lösungsmittel 8 entweder verdunstet, oder der Stent 18 wurde in ein Wasserbad eingetaucht. Die Figur 13 zeigt schematisch die infolge beider Varianten erzielte gleichmäßige Beschichtung des Stents 18 mit einem Wirkstoff 7. Die Figur 13 zeigt schematisch einen Schnitt durch einen Teil eines Gittergerüstes eines Stents 18. Auf der Oberfläche des Stents 18 befindet sich eine gleichmäßige Schicht aus einem Wirkstoff 7.

Eine weitere Variante ist in der Figur 14 schematisch dargestellt. Die Figur 14 zeigt ebenfalls einen Schnitt durch einen Teil des Gittergerüstes eines Stents 18. In dieser Variante wurde die Oberfläche des Stents 18 vor dem Benetzen mit einer Lösung des Wirkstoffes 7 in einem mit Wasser mischbaren Lösungsmittel 8 aufgeraut. Die aufgeraute Stentoberfläche ist mit der Bezugsziffer 19 in der Figur 14 gekennzeichnet. Nach dem Benetzen der aufgerauten Oberfläche 19 wurde wie in den vorangegangenen Varianten und Ausführungsbeispielen weiter verfahren. Die dadurch erzielte Beschichtung der Oberfläche 19 des Stents 18 ist in der Figur 14 schematisch gezeigt. Auf der aufgerauten Oberfläche 19 des Stents 18 hat sich im Ergebnis der verwendete Wirkstoff 7 gleichmäßig abgelagert.

In allen beschriebenen Ausführungsbeispielen und Ausführungsvarianten wird durch ein Entfernen von überflüssigem Wirkstoff und überflüssigem Lösungsmittel, beispielsweise durch Schleudern, Abschlagen oder schnelles Bewegen des Ballons des Ballonkatheters beziehungsweise des Stents, eine gleichmäßige Beschichtung der Oberfläche mit einem Wirkstoff erzielt. Dadurch kann insbesondere eine mögliche Bildung von Wirkstoffklumpen vermieden werden. Dies gewährleistet eine spätere gleichmäßige Medikamentenabgabe an das Gewebe im Körper des Patienten.

## Patentansprüche

1. Verfahren zur Erzeugung einer bioaktiven Oberfläche auf einer Endoprothese oder auf dem Ballon (3) eines Ballonkatheters (1), worin die Oberfläche der Endoprothese bzw. die Oberfläche des Ballons (3) mit einer Lösung (6) eines Wirkstoffs (7) benetzt wird, überflüssiger Wirkstoff (7) und Lösungsmittel (8) durch Bewegen der Endoprothese oder des Ballons (3) von der benetzten Oberfläche entfernt wird und das Lösungsmittel (8) von dem Wirkstoff (7) getrennt wird, wobei das Lösungsmittel (8) mit Wasser mischbar ist und der Wirkstoff (7) eine Löslichkeit von max. 0,9 mg/ml in destilliertem Wasser hat,
**dadurch gekennzeichnet, dass** das Lösungsmittel (8) von dem Wirkstoff (7) getrennt wird, indem die benetzte Endoprothese bzw. der benetzte Ballon (3) in Wasser (9) getaucht wird, wobei der in Wasser unlösliche Wirkstoff (7) ausfällt und sich teilweise auf der Oberfläche abscheidet.

2. Verfahren nach Anspruch 1 worin überflüssiger Wirkstoff (7) und Lösungsmittel (8) durch Abschleudern von der benetzten Oberfläche entfernt wird.

3. Verfahren nach Anspruch 1 oder 2, worin die Oberfläche der Endoprothese bzw. des Ballons (3) vor dem Benetzen vergrößert wird.

4. Verfahren nach Anspruch 3, worin die Oberfläche der Endoprothese bzw. des Ballons (3) mechanisch, thermisch oder chemisch, insbesondere durch Strukturieren oder Profilieren vergrößert wird.

5. Verfahren nach Anspruch 4, worin die Oberfläche der Endoprothese bzw. des Ballons (3) durch Aufrauen strukturiert oder profiliert wird.

6. Verfahren nach einem der Ansprüche 2 bis 5, worin durch das Vergrößern der Oberfläche der Endoprothese bzw. des Ballons (3) auf der Oberfläche Vertiefungen mit einer Tiefe von 5-50 µm und einer Breite von 5-50 µm erzeugt werden.

7. Verfahren nach einem der Ansprüche 1 bis 6, worin der verwendete Wirkstoff Tretinoin und/oder Tretinoinderivate und/oder Orphanrezeptoragonisten und/oder Elafinderivate und/oder Corticosteroide und/oder Steroidhormone und/oder Paclitaxel und/oder Taxol und/oder Taxolderivate und/oder Rapamune und/oder Tacrolimus und/oder hydrophobe Proteine und/oder zellproliferationsverändernde Substanzen umfaßt.

8. Verfahren nach Anspruch 7, worin als Steroidhormone Methylprednisolon, Dexamethason oder Ostradiol verwendet werden.

9. Verfahren nach einem der Ansprüche 1 bis 8, worin als Lösungsmittel Dimethylsulfoxid (DMSO), Dioxan, Dimethylformamid (DMF) oder Tetrahydrofuran (THE) verwendet wird.

10. Verfahren nach einem der Ansprüche 1 bis 9, worin die Oberfläche der Endoprothese bzw. des Ballons (3) durch Eintauchen, Besprühen oder Pipettieren benetzt wird.

11. Verfahren nach einem der Ansprüche 1 bis 10, worin die Oberfläche der Endoprothese bzw. des Ballons (3) vor der Erzeugung der bioaktiven Oberfläche mit einer Polymerschicht (12) funktionell beschichtet wird.

12. Verfahren nach Anspruch 11, worin die Dicke der funktionellen Polymerschicht (12) 10-1000 µm, insbesondere 200-400 µm beträgt.

13. Verfahren nach einem der Ansprüche 11 bis 12, worin die Oberfläche der Endoprothese bzw. des Ballons (3) vor der Erzeugung der bioaktiven Oberfläche mit Polyamino-p-xylylen-co-polyxylylen funktionell beschichtet wird.

14. Verfahren nach einem der Ansprüche 11 bis 13, worin zur Herstellung einer funktionelien Polymerschicht (12) aus den Ausgangsverbindungen der allgemeinen Strukturen (1), (2) und/oder (3) bei erhöhten Temperaturen und reduzierten Drücken Monomere in der Gasphase erzeugt und durch Abkühlung bei reduzierter Temperatur polymerisiert werden, mit: 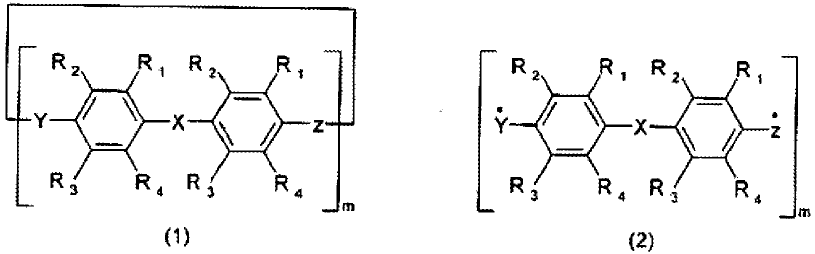 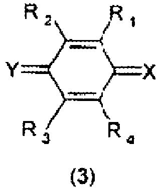 wobei:
R₁₂₃₄: jeweils, gleich oder verschieden voneinander, Wasserstoffatome, Halogenatome, Alkylgruppen oder substituierte Alkylgruppen, Arylgruppen oder substituierte Arylgruppen, organische Reste oder Radikale, Gruppen der allgemeinen Struktur CO(O-M-A), metallierte Gruppen, Hydroxylgruppen, Aminogruppen, Carboxylgruppen, Estergruppen, Ethergruppen, Säurehalogenidgruppen, Isocyanatgruppen, schwefelhaltige Gruppen, stickstoffhaltige Gruppen, phosphorhaltige Gruppen, siliziumhaltige Gruppen;
X,Y: Kohlenwasserstoffreste
m: Zahl der Wiederholungseinheiten = 1-20 und
wobei die zur Herstellung der Mononiere benötigten Temperaturen zwischen 500°C und 1000°C und die benötigten Drücke kleiner als 500 Pa sind.

15. Verfahren nach einem der Ansprüche 1 bis 14, worin als Endoprothese ein Stent (18) verwendet wird.

## Claims

1. Method for producing a bioactive surface on an endoprosthesis or on the balloon (3) of a balloon catheter (1), wherein the surface of the endoprosthesis or the surface of the balloon (3) is wetted with a solution (6) of an active ingredient (7), superfluous active ingredient (7) and solvent (8) is removed from the wetted surface by agitating the endoprosthesis or the balloon (3) and the solvent (8) is separated from the active ingredient (7), wherein the solvent (8) is miscible with water and the active ingredient (7) has a maximum solubility of 0.9 mg/ml in distilled water,
**characterized in that**,
the solvent (8) is separated from the active ingredient (7) by dipping the wetted endoprosthesis or the wetted balloon (3) in water (9), whereupon the water-insoluble active ingredient (7) precipitates and partially deposits on the surface.

2. Method according to Claim 1, wherein superfluous active ingredient (7) and solvent (8) is removed from the wetted surface by centrifugation.

3. Method according to Claim 1 or 2, wherein the surface area of the endoprosthesis or the balloon (3) is increased prior to wetting.

4. Method according to Claim 3, wherein the surface area of the endoprosthesis or the balloon (3) is increased by mechanical, thermal or chemical means, particularly by structuring or profiling.

5. Method according to Claim 4, wherein the surface of the endoprosthesis or the balloon (3) is structured or profiled by roughening.

6. Method according to any of Claims 2 to 5, wherein by increasing the surface area of the endoprosthesis or the balloon (3), indentations are generated on the surface having a depth of 5 - 50 µm and a width of 5 - 50 µm.

7. Method according to any of Claims 1 to 6, wherein the active ingredient used includes tretinoin or tretinoin derivatives and/or orphan receptor agonists and/or elafin derivatives and/or corticosteroids and/or steroid hormones and/or paclitaxel and/or taxol and/or taxol derivatives and/or rapamune and/or tacrolimus and/or hydrophobic proteins and/or cell proliferation altering substances.

8. Method according to Claim 7, wherein methylprednisolone, dexamethasone, or oestradiol are used as steroid hormones.

9. Method according to Claims 1 to 8, wherein dimethyl sulphoxide (DMSO), dioxane, dimethylformamide (DMF) or tetrahydrofuran (THF) is used as solvent.

10. Method according to any of Claims 1 to 9, wherein the surface of the endoprosthesis or the balloon (3) is wetted by dipping, spraying or pipetting.

11. Method according to any of Claims 1 to 10, wherein the surface of the endoprosthesis or the balloon (3) is functionally coated with a polymer layer (12) prior to producing the bioactive surface.

12. Method according to Claim 11, wherein the thickness of the functional polymer layer (12) is 10 - 1000 µm, particularly 200 - 400 µm.

13. Method according to either of Claims 11 or 12, wherein the surface of the endoprosthesis or balloon (3) is functionally coated with polyamino-p-xylylene-co-polyxylylene prior to producing the bioactive surface.

14. Method according to any of Claims 11 to 13, wherein for preparing a functional polymer layer (12) from the starting compounds of the general structures (1), (2) and/or (3) at elevated temperatures and reduced pressures, monomers are generated in the gas phase and are polymerized at reduced temperatures by cooling, with: 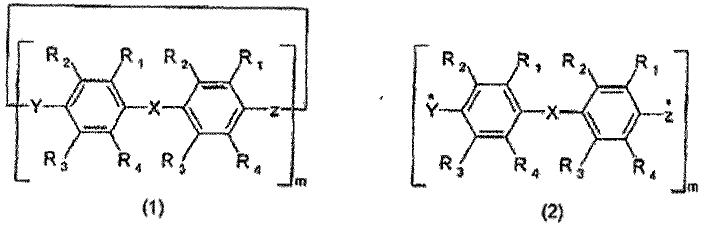 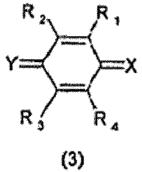 where:
R₁₂₃₄: in each case, the same or different, hydrogen atoms, halogen atoms, alkyl groups or substituted alkyl groups, organic residues or radicals, groups of the general structure CO(O-M-A), metallated groups, hydroxyl groups, amino groups, carboxyl groups, ester groups, ether groups, acid halide groups, isocyanate groups, sulphur-containing groups, nitrogen-containing groups, phosphorus-containing groups, silicon-containing groups;
X,Y: hydrocarbon residues
m: number of repeating units = 1-20 and
wherein the temperatures required for preparing the monomers are from 500°C to 1000°C and the pressures required are less than 500 Pa.

15. Method according to any of Claims 1 to 14, wherein a stent (18) is used as endoprosthesis.

## Revendications

1. Procédé pour générer une surface bioactive sur une endoprothèse ou sur le ballonnet (3) d'un cathéter (1) à ballonnet, dans lequel la surface de l'endoprothèse ou la surface du ballonnet (3) est mouillée par une solution (6) d'une substance active (7), la substance active (7) en excès et le solvant (8) sont éliminés de la surface mouillée par un mouvement de l'endoprothèse ou du ballonnet (3) et le solvant (8) est séparé de la substance active (7), le solvant (8) n'étant pas miscible avec l'eau et la substance active (7) présentant une solubilité d'au maximum 0,9 mg/ml dans l'eau distillée, **caractérisé en ce que** le solvant (8) est séparé de la substance active (7) **en ce que** l'endoprothèse mouillée ou le ballonnet (3) mouillé est plongé dans l'eau (9), la substance active (7) insoluble dans l'eau précipitant et se déposant en partie sur la surface.

2. Procédé selon la revendication 1, dans lequel la substance active (7) en excès et le solvant (8) sont éliminés par centrifugation de la surface mouillée.

3. Procédé selon la revendication 1 ou 2, dans lequel la surface de l'endoprothèse ou du ballonnet (3) est agrandie avant le mouillage.

4. Procédé selon la revendication 3, dans lequel la surface de l'endoprothèse ou du ballonnet (3) est agrandie mécaniquement, thermiquement ou chimiquement, en particulier par structuration ou profilage.

5. Procédé selon la revendication 4, dans lequel la surface de l'endoprothèse ou du ballonnet (3) est structurée ou profilée par rugosification.

6. Procédé selon l'une quelconque des revendications 2 à 5, dans lequel des creux d'une profondeur de 5-50 µm et d'une largeur de 5-50 µm sont générés à la surface par l'agrandissement de la surface de l'endoprothèse ou du ballonnet (3).

7. Procédé selon l'une quelconque des revendications 1 à 6, dans lequel la substance active utilisée est la trétinoïne et/ou des dérivés de la trétinoïne et/ou des agonistes des récepteurs orphelins et/ou des dérivés d'élafine et/ou des corticostéroïdes et/ou des hormones stéroïdiennes et/ou le paclitaxel et/ou le taxol et/ou des dérivés du taxol et/ou le rapamune et/ou le tacrolimus et/ou des protéines hydrophobes et/ou des substances modifiant la prolifération cellulaire.

8. Procédé selon la revendication 7, dans lequel on utilise, comme hormones stéroïdiennes, la méthylprednisolone, la dexaméthasone ou l'estradiol.

9. Procédé selon l'une quelconque des revendications 1 à 8, dans lequel on utilise, comme solvant, le diméthylsulfoxyde (DMSO), le dioxane, le diméthylformamide (DMF) ou le tétrahydrofuranne (THF).

10. Procédé selon l'une quelconque des revendications 1 à 9, dans lequel la surface de l'endoprothèse ou du ballonnet (3) est mouillée par immersion, pulvérisation ou pipetage.

11. Procédé selon l'une quelconque des revendications 1 à 10, dans lequel la surface de l'endoprothèse ou du ballonnet (3) est revêtue de manière fonctionnelle par une couche polymère (12) avant la génération de la surface bioactive.

12. Procédé selon la revendication 11, dans lequel l'épaisseur de la couche polymère (12) fonctionnelle est de 10-1000 µm, en particulier de 200-400 µm.

13. Procédé selon l'une quelconque des revendications 11 à 12, dans lequel la surface de l'endoprothèse ou du ballonnet (3) est revêtue de manière fonctionnelle par du polyamino-p-xylylène-co-polyxylylène avant la génération de la surface bioactive.

14. Procédé selon l'une quelconque des revendications 11 à 13, dans lequel, pour la réalisation d'une couche polymère (12) fonctionnelle à partir des composés de départ des structures générales (1), (2) et/ou (3) à des températures augmentées et des pressions réduites, des monomères sont générés dans la phase gazeuse et polymérisés par refroidissement à température réduits : 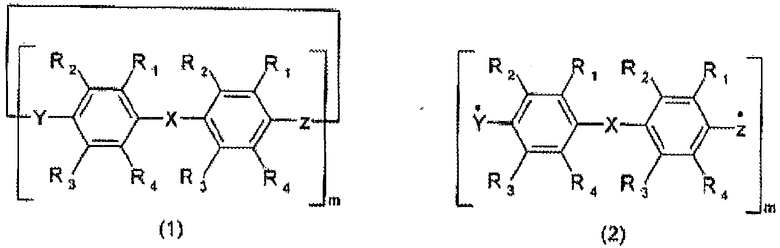 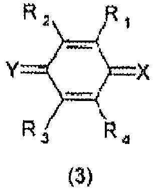 dans lesquelles :
R₁₂₃₄ : sont à chaque fois identiques ou différents les uns des autres et représentent des atomes d'hydrogène, des atomes d'halogène, des groupes alkyle ou des groupes alkyle substitués, des groupes aryle ou des groupes aryle substitués, des résidus ou des radicaux organiques, des groupes de structure générale CO(O-M-A), des groupes métallisés, des groupes hydroxyle, des groupes amino, des groupes carboxyle, des groupes ester, des groupes éther, des groupes halogénure d'acide, des groupes isocyanate, des groupes contenant du soufre, des groupes contenant de l'azote, des groupes contenant du phosphore, des groupes contenant du silicium ;
X,Y : représentent des résidus hydrocarbonés
m : représente le nombre d'unités récurrentes = 1-20 et
les températures nécessaires pour la préparation des monomères étant situées entre 500°C et 1000°C et les pressions nécessaires étant inférieures à 500 Pa.

15. Procédé selon l'une quelconque des revendications 1 à 14, dans lequel on utilise, comme endoprothèse, un stent (18).
